# EUROPEAN PATENT APPLICATION

(11) **EP 4 159 227 A1**
(43) Date of publication of application: **05.04.2023**
(21) Application number: 21200613.4
(22) Date of filing: 03.10.2021
(51) Int. Cl.: A61K 38/18, A61L 27/22, A61L 27/36, A61L 27/56, A61P 19/00, C07K 14/51, A61L 27/58, A61L 31/14, C12N 5/077

(54) **PHARMACEUTICAL COMPOSITION FOR PROMOTING OSTEOINDUCTION AND ANGIOGENESIS**

(71) Applicant: Jennissen, Herbert, 50858 Köln (DE)
(72) Inventor: Jennissen, Herbert, 50858 Köln (DE)
(74) Representative: Nobbe, Matthias

(57) **Abstract**

The present invention is directed to a pharmaceutical composition comprising glycosylated rhBMP-2 and non-glycosylated rhBMP-2 for promoting osteoinduction and angiogenesis, either in unary form for angiogenesis alone by non-glycosylated rhBMP-2 or in particular in a homologous binary growth factor composition for bone induction comprising glycosylated rhBMP-2 and non-glycosylated rhBMP-2.

## Description

The present invention is directed to a pharmaceutical composition comprising glycosylated rhBMP-2 and non-glycosylated rhBMP-2 for promoting osteoinduction and angiogenesis, either in unary form for angiogenesis alone by non-glycosylated rhBMP-2 or in particular in a homologous binary growth factor composition for bone induction comprising glycosylated rhBMP-2 and non-glycosylated rhBMP-2.

Human BMP-2 is a small, basic protein consisting of two identical polypeptide chains of 114 amino acids each (sequence molecular mass 12905 Da) covalently linked together to form the biologically active protein of 25.8 kDa. Mature BMP-2 is formed by processing from a larger precursor protein (396 amino acids).

Chinese hamster ovary (CHO) cells are commonly used mammalian host cells for industrial production of recombinant proteins. CHO cell expression systems produce a mixture of three rhBMP-2 isoforms (~ 36 kDa). A homodimer protein formed by the mature monomeric polypeptides, a homodimer, wherein each monomer comprises a 17-amino acid N-terminal extension to the mature monomeric polypeptide, and a heterodimer, wherein just one monomer comprises the 17-amino acid N-terminal extension.

The polypeptides produced in CHO cells are glycosylated (rhBMP-2_{CHO}). Various rhBMP-2 glycoforms are known.

The sequence of one polypeptide chain of the mature rhBMP-2 as derived from the proprotein is as follows.

(282)QAKHKQRK-RLKSSCKRHP-LYVDFSDVGW-NDWIVAPPGY-
HAFYCHGECP-FPLADHLNST-NHAIVQTLVN-SVNSKIPKAC-CVPTELSAIS-
MLYLDENEKV-VLKNYQDMVV-EGCGCR(396)

N56 of the mature protein, which corresponds to N338 of the proprotein, is a glycosylation site, known to be glycosylated in rhBMP-2_{CHO}.

The rhBMP-2 can also be expressed by *E*.*coli* comprising a BMP-2 expression plasmid that can produce the 114 amino acid polypeptide. Such prepared rh-BMP-2 is not glycosylated.

Due to the manufacturing process of the vector, the N-terminal amino acid may be altered, in particular with a serine preceding glutamine.

The mechanism of action of rhBMP-2 in patient therapy is still enigmatic, since the osteoinductive local dose must be 100-1000-fold higher in humans (e.g. 58 µM) than in animals (which respond to 10-20 nM concentrations) to be effective. Therefore, clinical applications of rhBMP-2 are restricted to only few indications (e.g. tibial fractures). Fundamental BMP-2 research in the areas of osseoinduction and angiogenesis is thus pertinent.

A biochemical solution according to the state of art for reducing these unphysiologically high therapeutic doses of rhBMP-2 could be a dose reduction based on the sequential (i.e. multimodal) application of several growth factors: e.g. of rhVEGF (38.3 kDa) blood vessel development) first, followed by rhBMP-2 (bone development) second. In this case it is of importance, that for both rhBMP-2 forms (E.coli and CHO) a nearly identical dissociation constant (rhBMP-2_{COL} (K_{D} = 3.2 ± 0.06 nM) and rhBMP-2_{CHO} (K_{D} = 3.1 ± 1.0 nM) has been determined in cell culture. In contrast the K_{D} for rhVEGF is over two orders of magnitude lower (K_{D} = 23 ± 3.5 pM).

In a co-culture system of human outgrowth endothelial cells (OEC) and human primary osteoblasts (pOB) angiogenesis can be induced by VEGF and by sonic hedgehog. Angiogenic properties have also been attributed to rhBMP-2 at ~100 ng/ml (4 nM). Recently, rhBMP-2 released from adsorbate biohybrids appeared to be directly involved in angiogenesis in the co-culture system OEC/pOB.

The disadvantage of the prior art process/products is that both osteoinduction and angiogenesis are not sufficiently achieved or can only be achieved with great effort by a precisely timed administration of various factors.

The problem to be solved can be seen in providing a product that overcomes the disadvantages known from the prior art. The task of the invention can thus inter alia be seen in providing a product that is able to induce both bone growth and blood vessel growth.

The problem is solved by a pharmaceutical composition comprising glycosylated rhBMP-2 and non-glycosylated rhBMP-2.

The glycosylation state of rhBMP-2 was found to affect the ability to induce bone growth or aniogenesis. It was found that non-glycosylated rhBMP-2 is not only biologically active for inducing bone but also for inducing angiogenesis i.e. microvessel-like structures. Glycosylated rhBMP-2 does not induce angiogenesis but is only osteoinductive. Interestingly, non-glycosylated rhBMP-2 was found to induce angiogenesis at very low concentrations (≤10 pM) known to be non-osteoinductive at this concentration.

Glycosylated rhBMP-2 is understood as a recombinant protein of human BMP-2 being glycosylated on at least one amino acid. Non-glycosylated rhBMP-2 is understood as a recombinant protein of human BMP-2 without being glycosylated.

Preferably, the glycosylated rhBMP-2 is rhBMP-2 produced in mammalian cells, such as CHO-cells. The non-glycosylated rhBMP-2 is preferably rhBMP-2 produced in bacteria, such as *E.coli.*

RhBMP-2, in the glycosylated form and in the non-glycosylated form, is preferably understood as a protein comprising two covalently linked polypeptide chains of 114 amino acids each with the amino acid sequence (1)QAKHKQRK-RLKSSCKRHP-LYVDFSDVGW-NDWIVAPPGY-HAFYCHGECP-FPLADHLNST-NHAIVQTLVN-SVNSKIPKAC-CVPTELSAIS-MLYLDENEKV-VLKNYQDMVV-EGCGCR(114), wherein the N-terminal Q can be preceded by S, in particular in the non-glycosylated form.

RhBMP-2 comprising one or two polypeptide chains having an N-terminal extension, such as the 17-amino acid N-terminal extension being obtained during the production in the CHO cells, is preferably also understood as rhBMP-2 according to the present invention.

The invention therefore embodies the combination of rhBMP-2 in the glyocsylated together with the nonglycosylated form to convey an additional angiogenic potential to the osteoinductive glycosylated form.

Preferably, the glycosylated rhBMP-2 of the pharmaceutical composition is a protein comprising two covalently linked polypeptide chains of 114 amino acids each with the amino acid sequence (1)QAKHKQRK-RLKSSCKRHP-LYVDFSDVGW-NDWIVAPPGY-HAFYCHGECP-FPLADHLNST-NHAIVQTLVN-SVNSKIPKAC-CVPTELSAIS-MLYLDENEKV-VLKNYQDMVV-EGCGCR(114) being glycosylated at N56.

Preferably the non-glycosylated rhBMP-2 of the pharmaceutical composition is a protein comprising two covalently linked polypeptide chains of each 114 amino acids with the amino acid sequence (1)QAKHKQRK-RLKSSCKRHP-LYVDFSDVGW-NDWIVAPPGY-HAFYCHGECP-FPLADHLNST-NHAIVQTLVN-SVNSKIPKAC-CVPTELSAIS-MLYLDENEKV-VLKNYQDMVV-EGCGCR(114) and a free N56.

An example for the combination of two growth factors is illustrated by the combination of rhBMP-2 with rhVEGF, i.e. a heterologous binary composition (see above). In that case a protein (rhVEGF) with a dissociation constant of K_{D} = 23 pM versus receptor and function is to be combined with a second protein (rhBMP-2) with a K_{D} = 3.2 nM versus receptor and function. These constants thus identify the respective physiological concentration ranges i.e. therapeutic margins, of both factors *in vivo.* Combining these proteins therefore requires a biological mixing ratio R reflecting the respective K_{D} values.

As initial value the molar ratio R of the two K_{D} values of 23 pM/3.2 nM can be taken, which results in R = 0.00719, for which a logarithmation is useful, resulting in log 0.00719 = Riog = -2.143. It is therefore practical to express the initial ratios of protein mixtures with highly different K_{D}-values as molar ratios in logarithmic form with the high affinity partner (i.e. lower K_{D}) in the numerator. Since receptor target saturation, shared receptor interference and interprotein interactions might occur, a range of R_{log}-values for the biological system has to be defined. In addition, the inactivaton and degradation of the growth factors in the applied tissues have to be considered.

RhBMP-2 in the glycosylated and non-glycosylated state can be expressed from the same DNA-template but in two different organisms (eukaryote chinese hamster ovary cells (CHO) and the prokaryote bacterium E. coli (COL). In this case, a homologous, binary protein mixture can be obtained for which a shared receptor interference and interprotein interactions at physiologsical concentrations should be minimal. However, inactivation and degradation rates *in vivo* are unknown and therefore require a wide range of Riog - values.

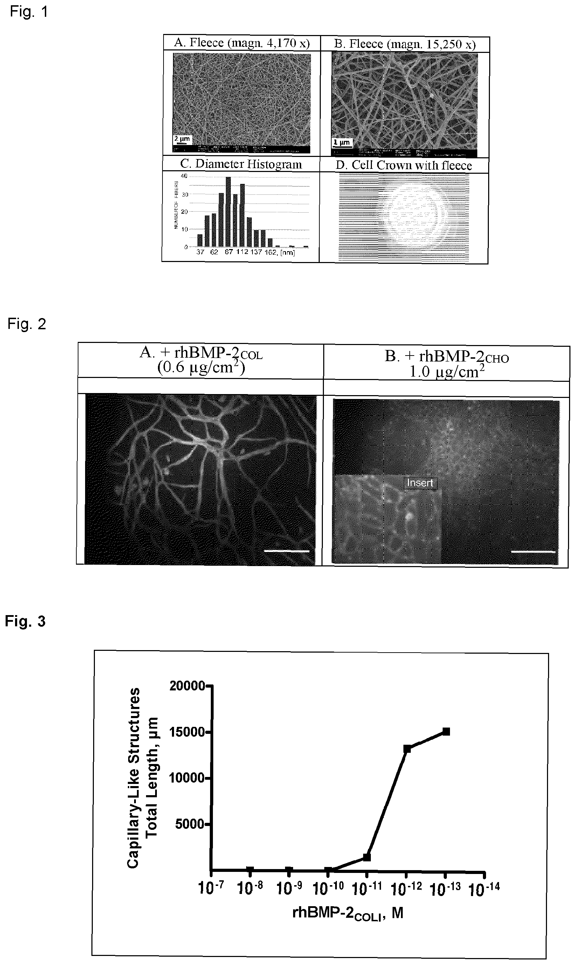

A picomolar to subpicomolar affinity of the angiogenic rhBMP-2_{COL} receptor interaction was found as shown in Fig. 3a. An estimate of K₀.₅ ( equivalent to K_{D} ) for induction of angiogenesis by rhBMP-2_{COL} results in a value of ~2 pM. For osteoinduction by rhBMP-2_{CHO} a value of 3.2 nM is obtained. Since these two values differ by three orders of magnitude identical receptors and shared receptors can be excluded with high probability. From these two K_{D}-values, the initial molar concentrations in solution and the initial molar ratio are derived for *in vitro* and *in vivo* conditions.. The minimal molar concentrations, below the initial values, are calculated to lie at 100 fM for rhBMP-2_{COL} and 100 pM for rhBMP-2_{CHO}-The initial molar ratio is calculated from the above K_{D}-values for the two rhBMP-2 forms to Riog = -3.2 as the midpoint for the range of molar ratios.

Since concentrations of rhBMP-2_{CHO} of up to 58 µM are required for osseoinduction in human therapy maximal molar concentrations of 58 µM for rhBMP-2_{CHO} and of 36 nM for rhBMP-2_{COL} of the initial ratio Riog = -3.2 are obtained. The preferred therapeutic ratios for humans at these concentrations again lie in the range of Riog = -1.5 to -6.0.

For the initial, minimal and maximal molar concentrations, the inventive molar ratios preferably lie in the range of Riog = -1.5 to -6.0. As estimated glycosylated rhBMP-2 and non-glycosylated rhBMP-2 are present in the initial molar ratio of Riog = -3.2 yielding a particularly preferable range of 1:10 (Riog = -1) to 1:1.000.000 (Riog = -6.0) and a most preferable range of 1:32 (R_{log} = -1.5), to 1:32.000 (R_{log} = -4.5). The molar concentration for the rhBMP-2_{COL} is therefore preferably in the range of below 10⁻¹¹ M, preferably below 10⁻¹² M or even lower, for administration preferably in an aqueous buffered solution and the respective concentration of the glycosylated rhBMP-2_{CHO} is in the respective ratio as expressed by the afore molar ratio.

In a preferred embodiment, the pharmaceutical composition comprises poly-(D,L)-lactide (PDLLA). PDLLA is a versatile material for BMP-2 delivery in physiological concentrations.

The pharmaceutical biohybrid composition can comprise a nanofiber fleece material, preferably a PDLLA nanofiber fleece material. PDLLA in the form of electrospun nanofiber fleeces (e.g. fiber ∅ 225-295 nm) is preferred. Proteins can either be immobilized inside the PDLLA nanofibers as an inclusate-biohybrid (IB) or on the surface of the nanofibers as an adsorbate-biohybrid (AB). In a preferred embodiment of the latter, the nanofiber fleece material of the above composition comprises 0.001 to 7.0 µg/cm², preferably 0.01 to 5.0 µg/cm² of non-glycosylated rhBMP-2. In a preferred embodiment the nanofiber fleece material of the composition comprises 0.5 to 50 µg/cm² of glycosylated rhBMP-2.

In a preferred embodiment, the pharmaceutical composition of the cellular co-culture system comprises human cells selected from endothelial cells, preferably outgrowth endothelial cells (OEC), osteoblasts, preferably human primary osteoblasts (pOB) or mixtures thereof. Preferred is a mixture of endothelial cells and of osteoblasts. Preferably the cells or mixture of cells are present on a fabric, such as the nanofiber fleece material mentioned above.

The pharmaceutical growth factor composition according to the present invention can be immobilized on various substrates. This pharmaceutical composition is preferably immobilized on an implant.

In a preferred embodiment, the non-glycosylated rhBMP-2 is obtained by deglycosylation of glycosylated rhBMP-2, preferably by means of endoglycosidase H.

The invention is further directed to the use of a pharmaceutical composition according to the present invention for coating an implant.

The invention is further directed to the use of a pharmaceutical composition according to the present invention for preparing an injection solution.

The invention is further directed to the use of a pharmaceutical composition comprising non-glycosylated rhBMP-2 and optionally further growth factors selected from the BMP-family, FGF-family, Interleukin family, TGF family and VEGF family, wherein the non-glycosylated rhBMP-2 is a protein comprising two covalently linked polypeptide chains of each 114 amino acids with the amino acid sequence QAKHKQRK-RLKSSCKRHP-LYVDFSDVGW-NDWIVAPPGY-HAFYCHGECP-FPLADHLNST-NHAIVQTLVN-SVNSKIPKAC-CVPTELSAIS-MLYLDENEKV-VLKNYQDMVV-EGCGCR for preparing an injection solution comprising the non-glycosylated rhBMP-2 in a concentration range of 10⁻¹⁰ to 10⁻¹⁶, preferably 10⁻¹¹ to 10⁻¹³mol/l.

rhBMP-2, in glycosylated and/or non-glycosylated form, can be used as an aqueous solution, preferably an aqueous salt solution, particularly preferably a buffered aqueous salt solution. The injection solution and/or the pharmaceutical composition therefore preferably comprise such a solution.

It was further found that a distinct receptor regulation prevents too many bloods vessels from being formed at high concentrations. The regulation corresponds to a high concentration cut-off regulation for an agonist/antagonist-receptor interaction, in which the same agonist of the receptor turns into an "antagonist" at high concentrations, i.e. the receptor function is "cut off" at high agonist concentrations. This regulation appears similar but not identical to a prior art mechanism called homologous desensitization. The distinct receptor regulation above appears significant also in respect to angiogenesis in other organs and tissues in general.

In the context of angiogenesis, the preparation of special carrier materials directed into releasing growth factors into cell cultures, tissues or organs in the pico- and subpicomolar ranges and especially of rhBMP2, specifically in the non-glycosylated form, is of special relevance: For rhBMP-2_{COL} it was determined that dissociation constant for immobilization onto the carriers of PDLLA (foamed or electrospun) lies at a K_{D} = 1.3 × 10⁻¹², demonstrating that at this dissociation constant sustained free concentrations of rhBMP-2_{COL} in the range of 10⁻¹¹ to 10⁻¹³ are created in the OEC/pOB co-culture medium. It is therefore of special relevance that the free concentration of rhBMP-2_{COL} in the respective medium is closely linked to the dissociation constant with which the BMP-2 has been immobilized on the surface.

Therefore, in preparing carriers for releasing free picomolar and subpicomolar concentrations of rhBMP-2, particularly of non-glycosylated rhBMP-2, into the medium, the proteins, such as rhBMP-2, have to be immobilized on the carrier with equivalent dissociations constants in the pico-/submolar range. Thus, such biohybrids then produce sustained rhBMP-2 concentrations, specifically in the non-glycosylated form, that generate blood vessels, particularly in an OEC/pOB co-culture. This procedure avoids high agonist (e.g. rhBMP-2_{COL}) concentrations, in which the agonist turns into a homologous antagonist inhibiting angiogenesis. Preferred dissociation constants for immobilization of the agonist on the carrier correspond to K_{D}-values of 10⁻⁹-10⁻¹⁶ mol/l, especially preferred K_{D}-values correspond to 10¹¹-10⁻¹⁵ mol/l.

The carrier may comprise or consist of biomaterials from the classes of metals, ceramics, polymers, and biomolecular materials, such as titanium, hydroxyapatite, polylactides and collagens comprising an implant. The rhBMP-2 or agonist to be released may be chemi- or physisorbed on the carrier or may be present in encapsulated PDLLA nanofiber form as long as the resulting preferred free concentrations in the range of 10⁻⁹-10⁻¹⁶ mol/I are obtained in the cell culture media or in the tissues or organs.

The present invention is further illustrated by the attached Figures and Tables and the Experimental part. As shown in the Figures:
Fig. 1 shows SEM Images of non-woven electrospun PDLLA Fleeces (A, B) with fiber Distribution (C) and mounted on a Cell Crown (D). The non-woven PDLLA nanofiber fleeces are shown at two different magnifications (Fig. 1A,B). A fiber diameter analysis yielded the distribution histogram (Fig. 1C) with a frequency maximum at a diameter of 87-100 nm. Since the paper-thin fleeces (discs ∅ 1.0 cm) are difficult to handle and can form rolls during incubation in the aqueous medium of the well plates, they were mounted on cell crowns for rhBMP-2 adsorption experiments and cell culture incubations (Fig. 1D).
Fig. 2 shows light microscopic images of the action of rhBMP-2_{COL} (A,) and of rhBMP-2_{CHO} (B ) after14 days in OEC/pOB co-culture system (magn. 10x).
   A. rhBMP-2_{COL}_Biohybrid (0.6 µg/cm²), Interconnected capillary-like structures (no cobblestones)
   B. rhBMP-2_{CHO}-Biohybrid (1 µg/cm²), Confluent cobblestone monolayer pattern, (no capillary-like structures); Insert: Additional magnification of original (Fig. 2B) ca. 3x The endothelial cells were immunofluorescently stained for CD31. Scale: 300 µm; B: inserted blow-up: ca. 3x.
Fig. 3 shows the Dose-Response-Curve of the induction of vessel-like structures by soluble rhBMP-2_{COL} in OEC/pOB co-culture system as a function of decreasing subpicomolar concentrations. Higher concentrations are inhibitory as pointed out above.

### Experimental part

Sterile lyophilized recombinant human bone morphogenetic protein 2, as rhBMP-2_{COL} (m = 26 kDa), expressed in E. coli (Morphoplant GmbH Bochum, D) and as rhBMP-2_{CHO} (*m*= 36 kDa) expressed in CHO-cells (InductOs^{®}, Medtronic BioPharma B.V., Heerlen, NL) were employed. The dose-response derived *biological equivalent activity* was K_{0.5} ~ 5-15 nM for both types of rhBMP-2. For determination of adsorbate loads on PDLLA fleeces rhBMP-2_{COL} was labeled with ¹²⁵| by the iodomonochloride method (initial specific radioactivity: 1.1 × 10⁹ cpm/mg) yielding ¹²⁵l-rhBMP-2, which was quantified in a γ-counter (Automatic γ-Counter 1480 Wizard 3, Wallac, FIN).

For adsorption experiments, ¹²⁵|-rhBMP-2_{COL} was diluted with cold native rhBMP-2_{COL} or rhBMP-2_{CHO} to 1-2 × 10⁷ cpm/mg. PDLLA nanofiber fleeces (membranes) were prepared on a custom-made electrospinning instrument. A spinning solution (7.5 wt% PDLLA) was prepared by adding 320 mg poly-DL-lactic acid (PDLLA; Resomer^{®} 207 S, Evonik Industries, Essen, D) to 2.96 g of hexafluoroisopropanol (HFIP; (abcr, Karlsruhe, D) and 0.74 g (ml) of distilled water, thus dissolving the PDLLA under stirring for 30 min at room temperature in a solvent mixture of 80 wt% HFIP and 20 g% distilled water to a final PDLLA concentration of 112.4 mg/ml. Because of the possibility of fleece scrolling and shrinking in solutions the PDLLA fleece disks (∅ 1.0 cm) were framed in cell-crown 24NX inserts (Scaffdex Oy, Tampere, FIN) (see Fig. 1) and inserted into 24-hole well plates. For preparation of adsorbate-biohybrids (AB) fleeces were incubated for 20 hours at room temperature under gentle shaking in BMP-2 solutions (10 µg/mL and 30 µg/mL) of rhBMP-2 in citrate buffer (5 mM sodium citrate, 14.6 mM sucrose, pH 4.5). Afterwards the protein solution was removed by aspiration, washed five times under shaking with Phosphate-buffered saline (DPBS, Gibco, Thermo Fischer, Darmstadt, D) at room temperature and air dried for 20 hours.

Primary human cells were obtained from excess tissue and their application was in accordance with the approved principle of informed consent. Human outgrowth endothelial cells (OECs) were isolated from peripheral blood buffy coats and expanded. Human primary osteoblasts (pOBs) were isolated from cancellous bone fragments from healthy donors. Co-culture experiments were performed with at least 3 different donors.

For co-culture experimentation, PDLLA fleeces on Cell Crowns were transferred to 24 well plates prior to cell seeding. Co-cultures consisting of endothelial cells (130.000/well) and primary osteoblasts (20.000/well) were seeded together on top of the different materials and were cultivated in endothelial cell growth medium (Promocell) depending on the experiment either for 7 days or for up to 14 days at 37°C in an atmosphere of 5% CO₂ and 95% air. For immunofluorescent staining, samples were permeabilized with 0.5 % Triton X/PBS and washed three times with PBS before they were incubated with mouse antihuman CD31 (1:40, Dako, MO 08223). The cells were examined using the fluorescence microscope (Nikon Eclipse TS 100) and stained for endothelial images (CD31). Microvessel-like structures were quantified using the image processing software NIS Elements (Nikon, Düsseldorf, Germany) either in total length (TL) of angogenic structures in [pixels]) and after calibration to microscale vessel length in µm (1 pixel ~ 0.45 µm). VEGF in cell culture supernatants was determined using DuoSet^{®} ELISA Development Systems (R&D Systems, Minneapolis, MN, USA) according to the manufacturers' protocol. Samples and standards were measured in triplicates using a microplate reader. Relative gene expression (RQ) of mRNA for the proteins osteopontin, alkaline phosphatase, and VEGF was determined using the Qiagen RNEasy Micro Kit together with the Omniscript RT kit according to the manufacturer's protocol (Qiagen, Hilden, Germany). Cell viability was tested by LDH Cytotoxicity Assay CyQUANT^{™} according to the manufacturer protocol (Invitrogen, Carlsbad, USA).

### Adsorption of ¹²⁵l-rhBMP-2

Radiotracer labelled ¹²⁵l-rhBMP-2 was adsorbed to the PDLLA nanofiber fleeces (Table 1) as described above yielding two biohybrid adsorbates each for the two rhBMP-2 forms. On placing the cell crowns with mounted nanofiber biohybrid adsorbates of rhBMP-2 into the well plates, it was observed that the cells not only adhered and grew on the fleece itself but also on the well bottom, indicating that the desorbed rhBMP-2 was diffusing into the well and inducing similar/identical cellular reactions not only on the fleece but also on the well bottom. Because of the well-bottom planarity and homogeneity it formed the basis for evaluation of the following experiments after 14 days in co-culture (Fig. 2).

**Table 1**

| **BMP-2** concentration | **Adsorbed Amount** | |
|---|---|---|
| | rhBMP-2_{COL} | rhBMP-2_{CHO} |
| 10 µg/mL | 0.6 µg/cm² | 1.0 µg/cm² |
| 30 µg/ml | 4.6 µg/cm² | 6.0 µg/cm² |

Table 1: Synthesis of adsorbate biohybrids of two forms of rhBMP-2 with PDLLA nanofiber fleeces for cell culture. The adsorption solution contained 10 and 30 µg/mL respectively. The amount adsorbed was determined by tracer detection with ¹²⁵l-rhBMP-2.

### Action of rhBMP-2_{COL} and of rhBMP-2_{CHO}

As shown in Fig. 2A the fleece with the low surface concentration of 0.6 µg/cm² adsorbed rhBMP-2_{COL} leads to an induction of interconnected angiogenic tube-like structures. In contrast the fleece with a high concentration of 4.6 µg/cm² only elicits a response in the range of the control without rhBMP-2 (not shown). Although rhBMP-2_{CHO} was biologically fully active and non-toxic in human use (Medtronic), no angiogenic activity, but only a classical confluent cobblestone monolayer pattern of the OEC colony in co-culture (Fig. 2B) was detected at both surface concentrations (Table 1). In fact this BMP-2_{CHO} also eliminated the residual spontaneous, endogenous angiogenic control activity (Fig. 2B).

### The quantitative results of the NIS-analysis

The quantitative results of the NIS-analysis are shown in Table 2

**Table 2**

| Angiogenic response induced by rhBMP-2 released from Biohybrid Fleeces ⁽¹⁾ | | |
|---|---|---|
| **rhBMP-2_{COL} Biohybrid Studies in Coculture** | | |
| ***Surface Concentration*** | ***Microvessel length µm*** | |
| | gross | net |
| Control: | | |
| 0 | 2530 | |
| rhBMP-2_{COL} | | |
| 0.6 µg/cm²: | 23958 | 21428 |
| 4.6 µg/cm²: | 4605 | 2075 |
| rhBMP-2_{CHO} | | |
| 1.0 µq/cm² | 0 | 0 |
| 6.0 µg/cm² | 0 | 0 |

| | | |
|---|---|---|
| ⁽¹⁾Microvessels (capillary-like structures) were determined by quantitative histologic staining (CD31; 1 pixel ~ 0.46 µm) . For further details see Fig. 2. High surface concentrations are inhibitory. | | |

At 0.6 µg/cm² a total angiogenic length of TL = 23,958 µm (Fig. 2A) . A ca. 7-fold increase in surface concentration to 4.6 µg/cm² (Fig. 3A) reduces the total angiogenic length to TL = 4,605 (20%). In the control a TL = 2,530 (10%) is found, i.e. rhBMP-2_{COL} leads to a ca. 10-fold increase in total length of the induced angiogenic structures versus the controls.

### Dependence of the appearance and total length of microvessel-like structures on the concentration of rhBMP-2_{COL}

In Fig. 3 and Table 3 a concentration-response experiment of the induction of angiogenic structures as a function of defined rhBMP-2_{COL} concentrations is shown. At concentrations of 2.6 pg/ml (0.1 pM) and 26 pg/ml (1.0 pM) there is a strong induction of angiogenic tube-like structures (~20,000 µm) decreasing to 10% at 260 pg/ml (10 pM) and disappearing at 2600 pg/ml (100 pM) with no further response at and above 2600 pg/ml (1000 pm). The results in Fig. 3 and Table 3 indicate a sub-picomolar high affinity of putative receptors binding to rhBMP-2_{COL}for angiogenesis.

**Table 3**

| Angiogenic response induced by decreasing concentrations of rhBMP-2_{COL} in solution⁽²⁾ | | |
|---|---|---|
| rhBMP-2_{COL} **Solution Studies in Coculture** | | |
| ***Concentrations*** | ***Microvessel length, µm*** | |
| | gross | net |
| Control (0 pM) | 5993 | |
| 1.1 pM | 21254 | 15261 |
| 1.0 pM | 19348 | 13355 |
| 10 pM | 7507 | 1514 |
| 100 pm | 0 | 0 |
| 1000 pm | 0 | 0 |

| | | |
|---|---|---|
| ⁽²⁾Microvessels (capillary-like structures) were determined by quantitative histologic qtaining (CD31; 1 pixel ~ 0.46 µm) . For further details see Fig. 2. High concentrations are inhibitory. | | |

For further validation of the above experiments additional parameters such as non-toxicity and cell viability by LDH and gene expression results via mRNA were obtained (not shown). LDH-release lies in the normal range. The osteogenic proteins alkaline phosphatase (ALP) and osteopontin (OP) are both strongly induced by the BMP-2_{COL}-biohybrids at 4.6 µg/cm². In contrast, at a 0.6 µg/cm², which is optimal for angiogenic stimulation by rhBMP-2_{COL} (Fig. 2A), there is no induction of ALP and VEGF and only a very low induction of osteopontin. These data provide strong evidence against the assumption that the angiogenic induction by rhBMP-2_{COL} proceeds via the pathway BMP-2/VEGF and provide strong evidence for a dual activity of rhBMP-2_{COL} released from the biohybrids by exhibiting a singular high angiogenic activity (Figs. 2-3) at low (0.6 µg/cm²) and singular potent osteogenic activity at high (4.6 µg/cm²) biohybrid surface concentrations respectively.

## Claims

1. Pharmaceutical composition for promoting osteoinduction and angiogenesis comprising glycosylated rhBMP-2 and non-glycosylated rhBMP-2.

2. Pharmaceutical composition according to claim 1, wherein the glycosylated rhBMP-2 is rhBMP-2 produced in mammalian cells, preferably CHO-cells, and the non-glycosylated rhBMP-2 is rhBMP-2 produced in bacteria, preferably *E.coli.*

3. Pharmaceutical composition according to claim 1 or 2, wherein the glycosylated rhBMP-2 is a protein comprising two covalently linked polypeptide chains of each 114 amino acids with the amino acid sequence QAKHKQRK-RLKSSCKRHP-LYVDFSDVGW-NDWIVAPPGY-HAFYCHGECP-FPLADHLNST-NHAIVQTLVN-SVNSKIPKAC-CVPTELSAIS-MLYLDENEKV-VLKNYQDMVV-EGCGCR being glycosylated at N56.

4. Pharmaceutical composition according to any one of claims 1, 2 or 3, wherein the non-glycosylated rhBMP-2 is a protein comprising two covalently linked polypeptide chains of each 114 amino acids with the amino acid sequence QAKHKQRK-RLKSSCKRHP-LYVDFSDVGW-NDWIVAPPGY-HAFYCHGECP-FPLADHLNST-NHAIVQTLVN-SVNSKIPKAC-CVPTELSAIS-MLYLDENEKV-VLKNYQDMVV-EGCGCR.

5. Pharmaceutical composition according to any one of claims 1 to 4, wherein the glycosylated rhBMP-2 and non-glycosylated rhBMP-2 are present in molar ratio in the range of R_{log} = -1,0 to R_{log} = -6.0, and in a more preferable range of R_{log} = -1.5 to R_{log} = -4.5.

6. Pharmaceutical composition according to any one of claims 1 to 5 comprising PDLLA.

7. Pharmaceutical composition according to any one of claims 1 to 6 comprising a nanofiber fleece material, preferably a PDLLA nanofiber fleece material.

8. Pharmaceutical composition according to any one of claims 1 to 7, wherein the nanofiber fleece material comprises 0,001 to 2,5 µg/cm², preferably 0,01 to 0,25 µg/cm² of non-glycosylated rhBMP-2.

9. Pharmaceutical composition according to any one of claims 1 to 8, wherein the nanofiber fleece material comprises 1 to 50 µg/cm² glycosylated rhBMP-2.

10. Pharmaceutical composition according to any one of claims 1 to 9 comprising human cells selected from endothelial cells, preferably outgrowth endothelial cells (OEC), osteoblasts, preferably human primary osteoblasts (pOB) or mixtures thereof, preferably a mixture of endothelial cells and of osteoblasts.

11. Pharmaceutical composition according to any one of claims 1 to 10, wherein the non-glycosylated rhBMP-2 is obtained by deglycosylation, preferably by means of an endoglycosidase, of glycosylated rhBMP-2.

12. Use of a pharmaceutical composition according to any one of claims 1 to 11 for coating an implant.

13. Use of a pharmaceutical composition according to the claims 1-11 for preparing an injection solution.

14. Use of a pharmaceutical composition comprising non-glycosylated rhBMP-2 and optionally further growth factors selected from BMP-family, FGF-family, Interleukin family, TGF family and VEGF family, wherein the non-glycosylated rhBMP-2 is a protein comprising two covalently linked polypeptide chains of 114 amino acids each with the amino acid sequence QAKHKQRK-RLKSSCKRHP-LYVDFSDVGW-NDWIVAPPGY-HAFYCHGECP-FPLADHLNST-NHAIVQTLVN-SVNSKIPKAC-CVPTELSAIS-MLYLDENEKV-VLKNYQDMVV-EGCGCR for preparing an injection solution comprising the non-glycosylated rhBMP-2 in a concentration range of 10⁻¹⁰ to 10^{-16,} preferably 10⁻¹¹ to 10⁻¹³mol/l.

15. Carrier made from a biocompatible material selected from metals, ceramics, polymers, biomolecular materials or combinations thereof, said carrier comprising a bioactive material selected from agonists and/or growth factors such as rhBMP-2, preferably non-glycosylated rhBMP2, on the carrier with a dissociation constant K_{D} 10⁻⁹ -10⁻¹⁶ mol/l, preferably 10⁻¹¹-10⁻¹⁵ mol/l, and/or said carrier being capable of releasing said bioactive material in the pico- and subpicomolar range for the induction of angiogenesis and other functions.
